# EUROPEAN PATENT SPECIFICATION

(11) **EP 2 827 941 B1**
(45) Date of publication and mention of the grant of the patent: **08.06.2016**
(21) Application number: 13714467.1
(22) Date of filing: 20.03.2013
(51) Int. Cl.: A61N 1/05, A61M 25/02

(54) **TORQUE LOCK ANCHOR AND METHODS AND DEVICES USING THE ANCHOR**
ANKER FÜR DREHMOMENTARRETIERUNG SOWIE VERFAHREN UND VORRICHTUNG ZUR VERWENDUNG DES ANKERS
ANCRE À VERROUILLAGE DE COUPLE ET PROCÉDÉS ET DISPOSITIFS UTILISANT L'ANCRE

(30) Priority: 22.03.2012 US 201213427679
(43) Date of publication of application: 28.01.2015
(73) Proprietor: Boston Scientific Neuromodulation Corporation, Valencia, CA 91355 (US)
(72) Inventor: PIANCA, Anne, Margaret, Santa Monica, CA 90403 (US); CHEN, Roger, Los Angeles, CA 90025 (US)
(74) Representative: Vossius & Partner Patentanwälte Rechtsanwälte mbB
(86) International application number: PCT/US2013/033180
(87) International publication number: WO 2013/142616

(56) References cited:
- WO-A1-98/33551
- WO-A1-2010/126853
- US-A- 5 376 108
- US-A1- 2011 060 395

## Description

### FIELD

The invention is directed to lead anchors for implantable devices, as well as the implantable devices themselves. The invention is also directed to lead anchors for implantable spinal cord stimulators, as well as the implantable spinal cord stimulators.

### BACKGROUND

Spinal cord stimulation is a well accepted clinical method for reducing pain in certain populations of patients. Implantable stimulation devices have been developed to provide therapy for a variety of treatments. For example, implantable stimulation devices can be used to stimulate nerves, such as the spinal cord, muscles, or other tissue. An implantable stimulation device typically includes an implanted control module (with a pulse generator), a lead, and an array of stimulator electrodes. The stimulator electrodes are implanted in contact with or near the nerves, muscles, or other tissue to be stimulated. The pulse generator in the control module generates electrical pulses that are delivered by the electrodes to body tissue. As an example, electrical pulses can be provided to the dorsal column fibers within the spinal cord to provide spinal cord stimulation.

The stimulator electrodes are coupled to the control module by the lead and the control module is implanted elsewhere in the body, for example, in a subcutaneous pocket. The lead is often anchored at one or more places in the body to prevent or reduce movement of the lead or stimulator electrodes within the body which could damage tissue, move the stimulator electrodes out of the desired position, or interrupt the connection between the stimulator electrodes and the control module.

WO 2010/126853 A1 discloses a lead anchor including a body defining a lead lumen having a first opening and a second opening through which a lead can pass. The body further defines a transverse lumen that intersects the lead lumen. An exterior member is disposed around at least a portion of the body. The exterior member is formed of a biocompatible material. A fastener anchors the lead to the body through the transverse lumen by deforming a portion of the lead. The transverse lumen is configured and arranged to receive the fastener. At least one suture element is defined by the exterior member and is configured and arranged for receiving a suture to suture the lead anchor to patient tissue.

Many conventional lead anchors do not sufficiently grip the lead to keep the lead in place. According to recent studies, lead migration occurs in approximately 13% of cases. Additional studies suggest that electrode migration may be the most common reason for failure to maintain long-term pain control with spinal cord stimulation. Other problems associated with lead migration include lead breakage, and loose connection.

Yet another problem associated with conventional lead anchors is that they are typically anchored at one or more places in the body to prevent or reduce movement of the lead by securing the anchor using sutures. These anchors are highly dependent on suturing technique and lead to variable holding forces. Depending on the physician, the suturing of the lead to the anchor may be too loose or too tight. Furthermore, the increased suturing may lead to an increased operation time with a greater risk of infection.

### BRIEF SUMMARY

In one embodiment, a lead anchor includes a body having an outer surface, a top end, a front side, a first end, and a second end opposite to the first end. The body defines a lead slot configured and arranged to receive a lead. The lead slot defines an elongated opening extending along the front side of the body from the first end to the second end. A transverse lumen extends from the top end of the body and intersects the lead slot. An exterior member is disposed around at least a portion of the body. The exterior member is formed of a biocompatible material. A fastener is disposed in the transverse lumen. The fastener is configured and arranged for fastening the received lead to the lead anchor by deforming a portion of the lead.

An exemplary method of implanting an implantable stimulation device includes implanting an electrode array of a lead near patient tissue to be stimulated. A lead anchor is provided. The lead anchor includes a body having an outer surface, a top end, a front side, a first end, and a second end opposite to the first end. The body defines a lead slot with an elongated opening extending along the front side of the body from the first end to the second end, and a transverse lumen extending from the top end of the body and intersecting the lead slot. An exterior member is disposed around at least a portion of the body. The exterior member is formed of a biocompatible material. A fastener is disposed in the transverse lumen. A portion of the lead is slid into the lead slot such that the lead anchor slides perpendicular to a longitudinal length of the lead. The fastener is tightened to secure the lead anchor to the lead. Tightening the fastener deforms a portion of the lead.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system, according to the invention;
FIG. 2 is a schematic view of another embodiment of an electrical stimulation system, according to the invention;
FIG. 3A is a schematic view of one embodiment of a proximal portion of a lead and a control module of an electrical stimulation system, according to the invention;
FIG. 3B is a schematic view of one embodiment of a proximal portion of a lead and a lead extension of an electrical stimulation system, according to the invention;
FIG. 4A is a schematic cross-sectional view of one embodiment of a torque lead anchor with a pressure plate according to the invention;
FIG. 4B is a schematic cross-sectional view of another embodiment of a torque lead anchor with a pressure plate according to the invention;
FIG. 4C is a schematic side view of the torque lead anchor of Figure 4B, according to the invention;
FIG. 4D is a schematic cross-sectional view of another embodiment of a torque lead anchor with a septum, according to the invention;
FIG. 5 is a schematic cross-sectional view of the torque lead anchor of Figure 4A after a lead has been inserted in the lead anchor according to the invention;
FIG. 6 is a schematic cross-sectional view of one embodiment of a torque lead anchor with a sleeve according to the invention;
FIG. 7 is a schematic cross-sectional view of the torque lead anchor of Figure 6 after a lead has been inserted in the lead anchor according to the invention;
FIG. 8A is a schematic perspective view of a portion of another embodiment of a lead anchor according to the invention;
FIG. 8B is a schematic side view of the portion of the lead anchor of Figure 8A according to the invention;
FIG. 8C is a schematic front view of the portion of the lead anchor of Figure 8A according to the invention;
FIG. 8D is a schematic top view of the portion of the lead anchor of Figure 8A according to the invention;
FIG. 9A is a schematic perspective view of a portion of another embodiment of a lead anchor according to the invention;
FIG. 9B is a schematic side view of the portion of the lead anchor of Figure 9A according to the invention;
FIG. 9C is a schematic front view of the portion of the lead anchor of Figure 9A according to the invention;
FIG. 9D is a schematic top view of the portion of the lead anchor of Figure 9A according to the invention;
FIG. 10 is a schematic perspective view of the lead anchor of Figure 9A attached to a lead according to the invention;
FIG. 11 is a schematic perspective view of the lead anchor of Figure 9A and a tool according the invention;
FIG. 12 is a schematic overview of one embodiment of components of an electrical stimulation system, including an electronic subassembly disposed within a control module, according to the invention;
FIG. 13A is a schematic perspective view of another embodiment of a lead anchor, according to the invention;
FIG. 13B is a schematic bottom view of the lead anchor of Figure 13A, according to the invention;
FIG. 13C is a schematic perspective view of a body of the lead anchor of Figure 13A, according to the invention;
FIG. 13D is a schematic cross-sectional view of an exterior member of the lead anchor of Figure 13A, according to the invention;
FIG. 14A is a schematic end view of another embodiment of a lead anchor with a lead slot defined along a front side of the lead anchor for receiving a lead, according to the invention;
FIG. 14B is a schematic end view of the lead anchor of FIG. 14A, the lead anchor including an exterior member disposed over a body of the lead anchor, according to the invention;
FIG. 14C is a front side view of the lead anchor of FIG. 14A with a lead disposed in a lead slot of the lead anchor, according to the invention;
FIG. 15 is a schematic side view of one embodiment of a lead anchor with one or more staple tabs extending from the lead anchor, and staple eyelets defined in the staple tabs for stapling the lead anchor to the patient tissue;
FIG. 16A is a schematic side view of one embodiment of a lead anchor with an elongated, tapered distal end;
FIG. 16B is a schematic side view of one embodiment of the lead anchor of FIG. 16A with ridges disposed on an elongated, tapered distal end of the lead anchor; and
FIG. 16C is a schematic side view of another embodiment of the lead anchor with first ridges disposed on an elongated, tapered distal end of the lead anchor and second ridges disposed on an elongated, tapered proximal end of the lead anchor.

### DETAILED DESCRIPTION

The present invention is directed to the area of lead anchors used with elongate implantable devices such as spinal cord leads, cardiac pacing leads or catheters, implantable devices or systems containing the lead anchors, methods of use and manufacture of lead anchors and implantable devices. In addition, the invention is directed to lead anchors for implantable spinal cord stimulators, as well as the stimulators themselves and methods of use and manufacture of the lead anchors and spinal cord stimulators.

Suitable implantable electrical stimulation systems include, but are not limited to, an electrode lead ("lead") with one or more electrodes disposed on a distal end of the lead and one or more terminals disposed on one or more proximal ends of the lead. Leads include, for example, percutaneous leads, paddle leads, and cuff leads. Examples of electrical stimulation systems with leads are found in, for example, U.S. Patents Nos. 6,181,969; 6,516,227; 6,609,029; 6,609,032; and 6,741,892; 7,244,150; 7,672,734;7,761,165; 7,949,395; 7,974,706; U.S. Patent Application Publication Nos. 2005/0165465 A1; 2007/0150036 A1; and 2007/0219595 A1.

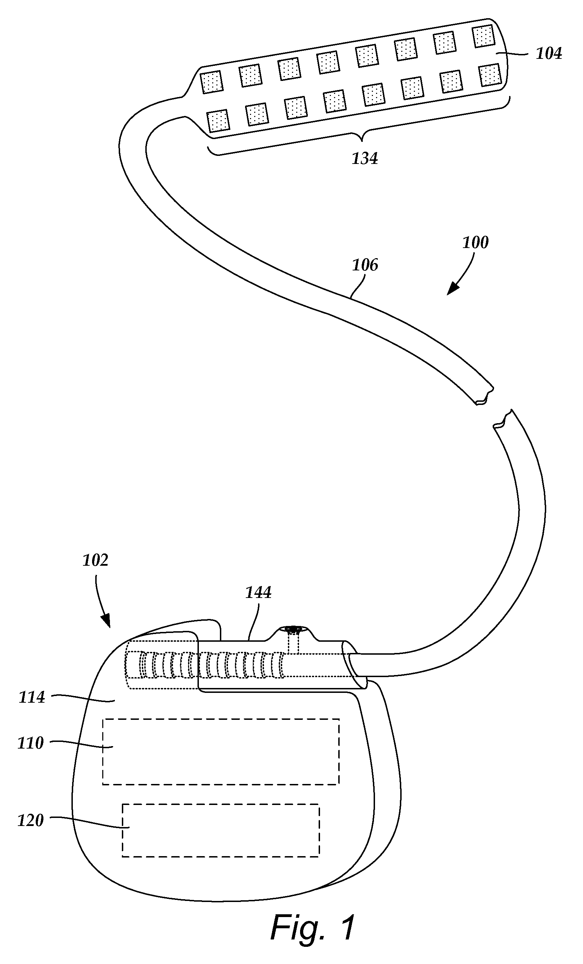

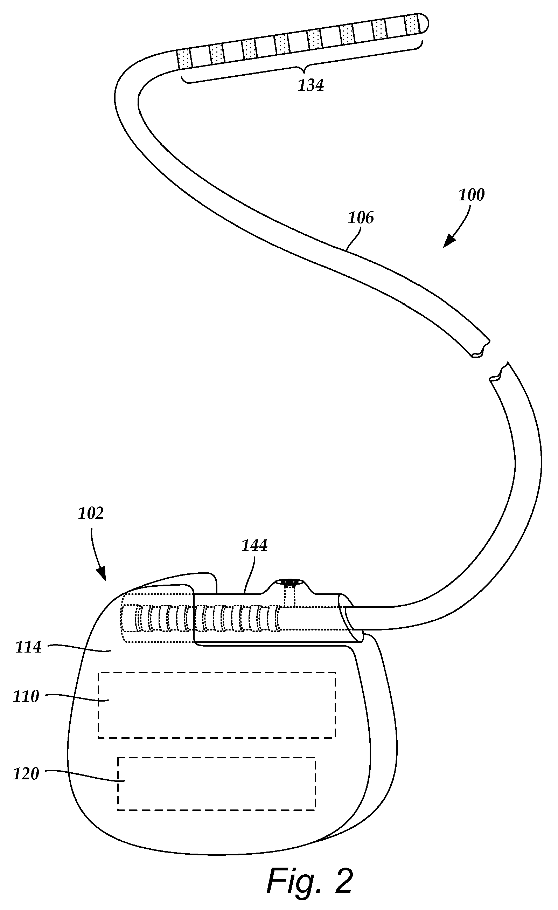

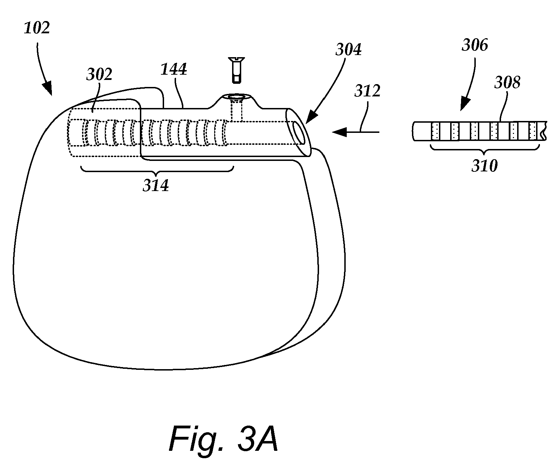

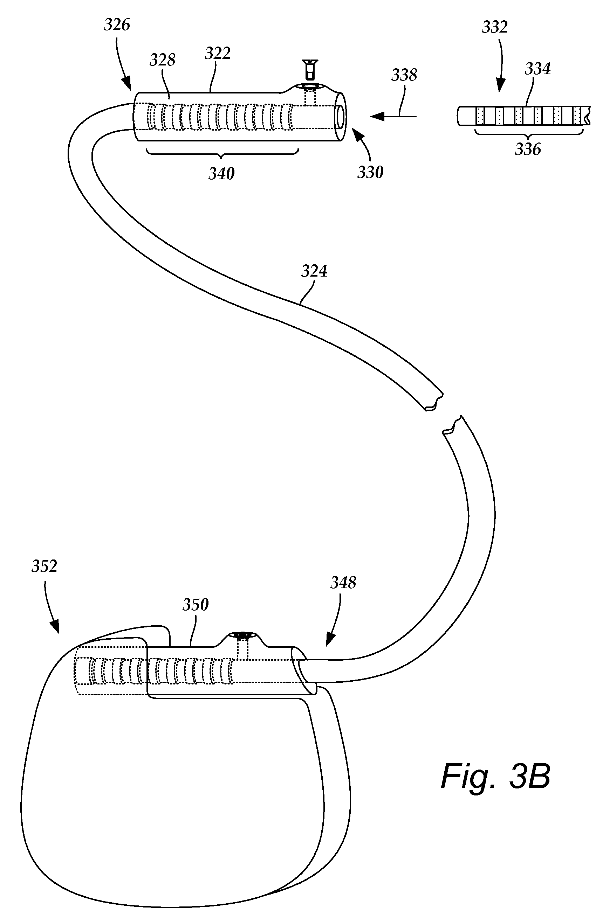

Figure 1 illustrates schematically one embodiment of an electrical stimulation system 100. The electrical stimulation system includes a control module *(e.g.,* a stimulator or pulse generator) 102, a paddle body 104, and at least one lead body 106 coupling the control module 102 to the paddle body 104. The paddle body 104 and the one or more lead bodies 106 form a lead. The paddle body 104 typically includes an array of electrodes 134. The control module 102 typically includes an electronic subassembly 110 and an optional power source 120 disposed in a sealed housing 114. The control module 102 typically includes a connector 144 (Figure 2 and 3A, see also 322 and 350 of Figure 3B) into which the proximal end of the one or more lead bodies 106 can be plugged to make an electrical connection via connector contacts on the control module 102 and terminals *(e.g.,* 310 in Figure 3A and 336 of Figure 3B) on each of the one or more lead bodies 106. It will be understood that the electrical stimulation system can include more, fewer, or different components and can have a variety of different configurations including those configurations disclosed in the electrical stimulation system references cited herein. For example, instead of a paddle body 104, the electrodes 134 can be disposed in an array at or near the distal end of the lead body 106 forming a percutaneous lead, as illustrated in Figure 2. A percutaneous lead may be isodiametric along the length of the lead. In addition, one or more lead extensions 312 (see Figure 3B) can be disposed between the one or more lead bodies 106 and the control module 102 to extend the distance between the one or more lead bodies 106 and the control module 102 of the embodiments shown in Figures 1 and 2.

The electrical stimulation system or components of the electrical stimulation system, including one or more of the lead bodies 106, the paddle body 104, and the control module 102, are typically implanted into the body of a patient. The electrical stimulation system can be used for a variety of applications including, but not limited to, brain stimulation, neural stimulation, spinal cord stimulation, muscle stimulation, and the like.

The electrodes 134 can be formed using any conductive, biocompatible material. Examples of suitable materials include metals, alloys, conductive polymers, conductive carbon, and the like, as well as combinations thereof. The number of electrodes 134 in the array of electrodes 134 may vary. For example, there can be two, four, six, eight, ten, twelve, fourteen, sixteen, or more electrodes 134. As will be recognized, other numbers of electrodes 134 may also be used.

The electrodes of the paddle body 104 or one or more lead bodies 106 are typically disposed in, or separated by, a non-conductive, biocompatible material including, for example, silicone, polyurethane, polyetheretherketone, epoxy, and the like or combinations thereof. The paddle body 104 and one or more lead bodies 106 may be formed in the desired shape by any process including, for example, molding (including injection molding), casting, and the like. Electrodes and connecting wires can be disposed onto or within a paddle body either prior to or subsequent to a molding or casting process. The non-conductive material typically extends from the distal end of the lead to the proximal end of each of the one or more lead bodies 106. The non-conductive, biocompatible material of the paddle body 104 and the one or more lead bodies 106 may be the same or different. The paddle body 104 and the one or more lead bodies 106 may be a unitary structure or can be formed as two separate structures that are permanently or detachably coupled together.

Terminals (e.g., 310 in Figure 3A and 336 of Figure 3B) are typically disposed at the proximal end of the one or more lead bodies 106 for connection to corresponding connector contacts *(e.g.,* 314 in Figure 3A and 340 of Figure 3B) in connectors *(e.g.,* 144 in Figures 1-3A and 322 and 350 of Figure 3B) disposed on, for example, the control module 102 (or to other devices, such as connector contacts on a lead extension, an operating room cable, or an adaptor). Conductive wires ("conductors") (not shown) extend from the terminals (e.g., 310 in Figure 3A and 336 of Figure 3B) to the electrodes 134. Typically, one or more electrodes 134 are electrically coupled to a terminal *(e.g.,* 310 in Figure 3A and 336 of Figure 3B). In some embodiments, each terminal *(e.g.,* 310 in Figure 3A and 336 of Figure 3B) is only connected to one electrode 134. The conductors may be embedded in the non-conductive material of the lead or can be disposed in one or more lumens (not shown) extending along the lead. In some embodiments, there is an individual lumen for each conductor. In other embodiments, two or more conductors may extend through a lumen. There may also be one or more lumens (not shown) that open at, or near, the proximal end of the lead, for example, for inserting a stylet rod to facilitate placement of the lead within a body of a patient. Additionally, there may also be one or more lumens (not shown) that open at, or near, the distal end of the lead, for example, for infusion of drugs or medication into the site of implantation of the paddle body 104. In at least one embodiment, the one or more lumens may be flushed continually, or on a regular basis, with saline, epidural fluid, or the like. In at least some embodiments, the one or more lumens can be permanently or removably sealable at the distal end.

In at least some embodiments, leads are coupled to connectors disposed on control modules. In Figure 3A, a lead 308 is shown configured and arranged for insertion to the control module 102. The connector 144 includes a connector housing 302. The connector housing 302 defines at least one port 304 into which a proximal end 306 of a lead 308 with terminals 310 can be inserted, as shown by directional arrow 312. The connector housing 302 also includes a plurality of connector contacts 314 for each port 304. When the lead 308 is inserted into the port 304, the connector contacts 314 can be aligned with the terminals 310 on the lead 308 to electrically couple the control module 102 to the electrodes (134 of Figure 1) disposed at a distal end of the lead 308. Examples of connectors in control modules are found in, for example, U.S. Patent No. 7,244,150 and U.S. Patent Application Publication No. 2008/0071320 A1.

In Figure 3B, a connector 322 is disposed on a lead extension 324. The connector 322 is shown disposed at a distal end 326 of the lead extension 324. The connector 322 includes a connector housing 328. The connector housing 328 defines at least one port 330 into which a proximal end 332 of a lead 334 with terminals 336 can be inserted, as shown by directional arrow 338. The connector housing 328 also includes a plurality of connector contacts 340. When the lead 334 is inserted into the port 330, the connector contacts 340 disposed in the connector housing 328 can be aligned with the terminals 336 on the lead 334 to electrically couple the lead extension 324 to the electrodes (134 of Figure 1) disposed at a distal end (not shown) of the lead 334.

In at least some embodiments, the proximal end of a lead extension is similarly configured and arranged as a proximal end of a lead. The lead extension 324 may include a plurality of conductors (not shown) that electrically couple the connector contacts 340 to a proximal end 348 of the lead extension 324 that is opposite to the distal end 326. In at least some embodiments, the conductors disposed in the lead extension 324 can be electrically coupled to a plurality of terminals (not shown) disposed on the proximal end 348 of the lead extension 324. In at least some embodiments, the proximal end 348 of the lead extension 324 is configured and arranged for insertion into a connector disposed in another lead extension. In other embodiments, the proximal end 348 of the lead extension 324 is configured and arranged for insertion into a connector disposed in a control module. As an example, in Figure 3B the proximal end 348 of the lead extension 324 is inserted into a connector 350 disposed in a control module 352.

A lead anchor can be used in an implantable device, such as an implantable spinal cord stimulator, to anchor a lead connecting a control module to an electrode array. The lead anchor includes a fastener, which may be tightened to hold the lead. In at least some embodiments, the lead anchor applies gentle compression to the lead to hold the lead in place.

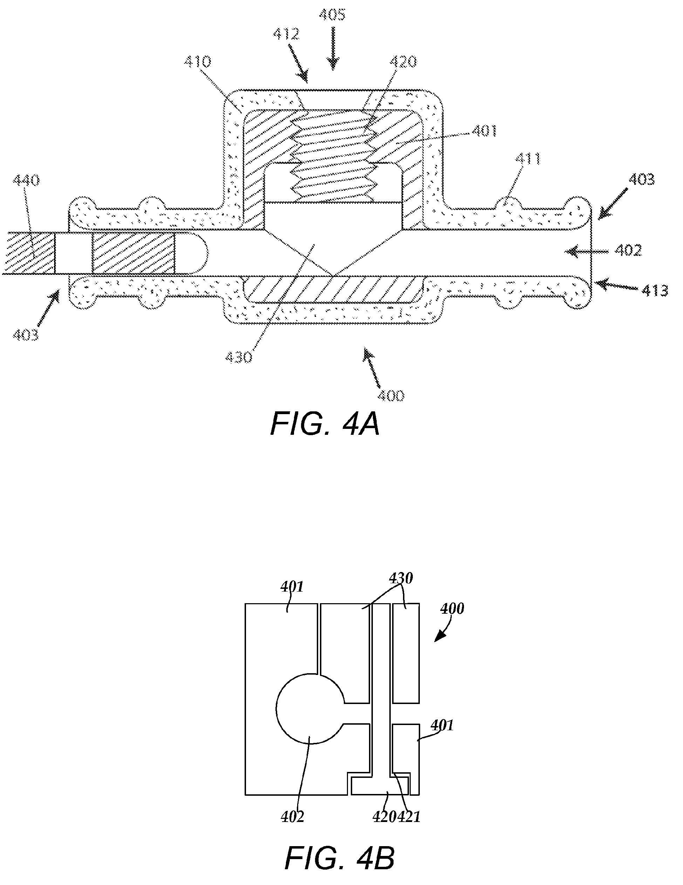

FIG. 4A is a schematic cross-sectional view of one embodiment of a torque lead anchor 400. As shown in FIG. 4A, the torque lead anchor 400 includes a body 401 and an exterior member 410. The body 401 may be made of a metal, such as titanium, nickel, aluminum, stainless steel, copper, gold, silver, platinum and alloys thereof or any other biocompatible metal, or a rigid plastic or polymer material. The exterior member 410 may be formed of any biocompatible material such as plastics and polymers including, but not limited to, silicone, polyvinyl chloride, fluoropolymers, polyurethane, polycarbonate, acrylic compounds, thermoplastic polyesters, polypropylene, low-density polyethylenes, and other thermoplastic elastomers. In some embodiments, the exterior member 410 is made of silicone. In some embodiments, the exterior member and the body are made of the same material. In some embodiments, the exterior member and the body are unitary.

Furthermore, it may be useful for any or all parts of the lead anchor to be made of a material that is radiopaque, so that it is visible under fluoroscopy or other forms of x-ray diagnosis. In some embodiments, the body or the exterior member is radiopaque so as to allow the lead anchor to be readily identified under fluoroscopy or other forms of x-ray diagnosis. The lead itself may also be radiopaque.

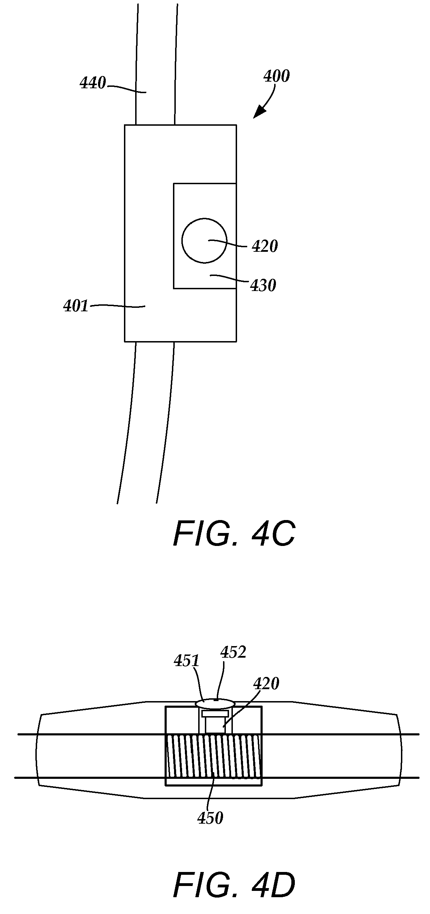

The body 401 contains a lead lumen 402 through which a lead 440 may pass. The lead lumen 402 has a first opening 403 and a second opening 404 for insertion of the lead. The lead lumen 402 may have a cross-section that is substantially circular as it extends from the first opening 403 to the second opening 404. It is contemplated that the lead lumen 402 may also have a cross-section in the shape of a triangle, a square, an ovoid, or any other suitable shape that is large enough to house the lead 440. In some embodiments the lead lumen 402 may be defined so that the lead 440 passes along a straight path through the center of the body 401. Conversely the lead lumen 402 may be defined so that the lead 440 passes at an angled path through the body 401. In some embodiments, the lead lumen 402 is defined as a curved path through the body 401. In some embodiments, the body 401 contains more than one lead lumen so that the lead anchor 400 is able to house more than one lead. The opening may be a friction fit with the lead or can be large enough to allow the lead to pass through freely. In some embodiments, the lead lumen 402 is formed of a tapped and reamed through lumen. As seen in Figure 4D, the lead lumen 402 may also include ridges 450, which may be concentric, for better engagement with the lead 440. The lead lumen 402 may alternatively define an interior thread or another pattern for better engagement with the lead 440.

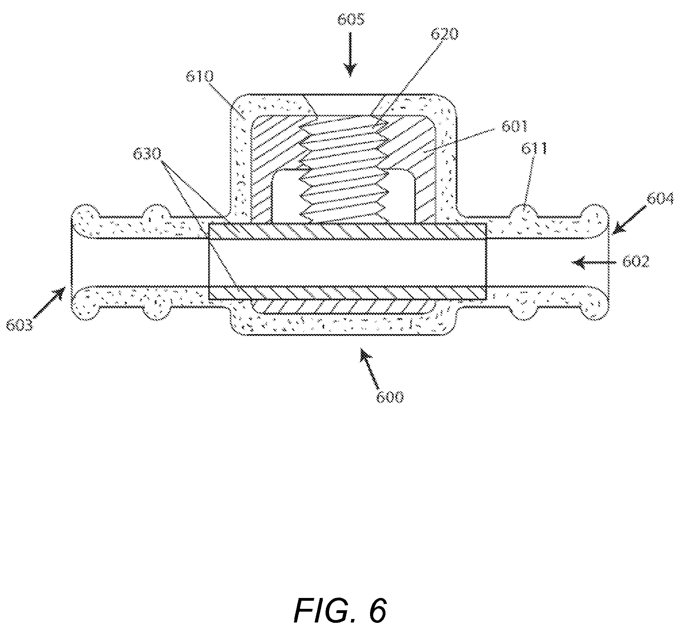

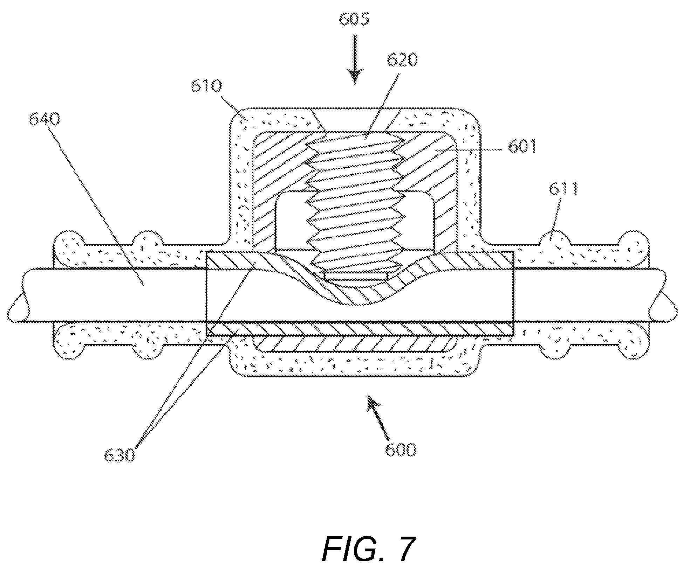

The body 401 further defines a transverse lumen 405 for accepting a fastener 420. The transverse lumen 405 may have a cross-section that is substantially circular. In other embodiments, the body 401 defines a transverse lumen 405 with a cross-section in the shape of a triangle, a square, an ovoid, or any other suitable shape that is capable of housing the fastener 420. In some embodiments, the transverse lumen 405 is positioned perpendicular to the central axis of the lead lumen 402. In other embodiments, the transverse lumen 405 may be defined so that the fastener 420 engages a lead 440 within the lead lumen 402 at a 15, 30, or 45 degree angle or any other suitable angle with respect to the central axis of the lumen. In some embodiments, the transverse lumen 405 intersects the lead lumen 402 and extends through it so that a cross-shaped void is formed through the body 401. In at least some embodiments, the transverse lumen 405 merges with the lead lumen 402 but does not extend through it, so that the cross-section of the body 401 defines a T-shaped bore. In at least some embodiments, the two lumens intersect with a sleeve or a plate disposed between the two (see, e.g., Figures 6 and 7). In embodiments with multiple lead lumens, the body 401 may define more than one transverse lumen for accepting a plurality of fasteners. Additionally, the body 401 may define a transverse lumen 405 having a thread, groove, crease, channel, duct or rib for facilitating or accepting the fastener 420.

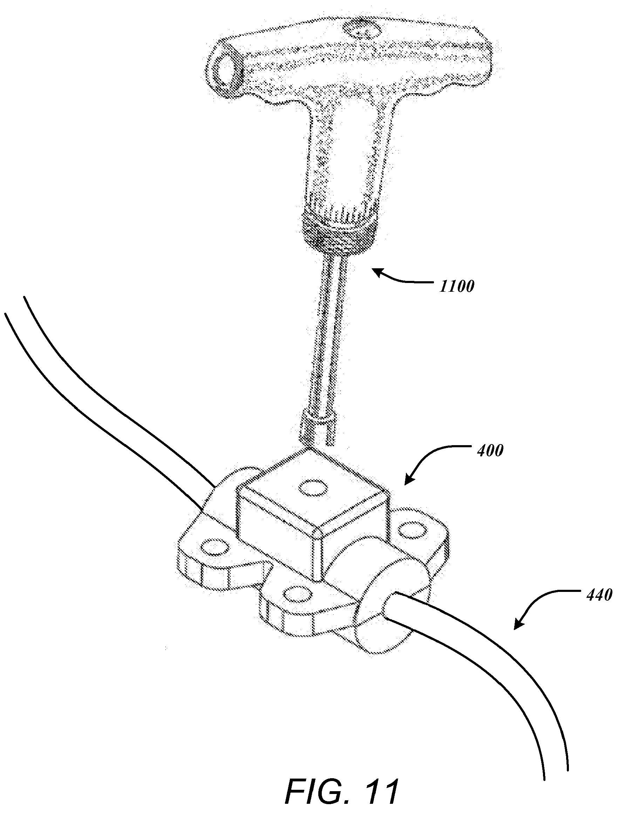

The fastener 420 may be, for example, a pin, clamp, latch, lug, nail, bolt, dowel, rod, rivet, screw or any combination thereof or any other suitable item for engaging and anchoring the lead. The fastener 420 may engage or couple to the lead anchor 400 by any method such as, for example, tightening, screwing or pushing. In some embodiments, the fastener 420 is a set screw with a thread to be received by the transverse lumen 405 of the body 401. The set screw may be tightened through the use of a torque limiting tool 1100 (see Figure 11), which will be described in greater detail below. As the fastener 420 engages the body 401 through the transverse lumen 405, it is brought closer to the lead lumen 402.

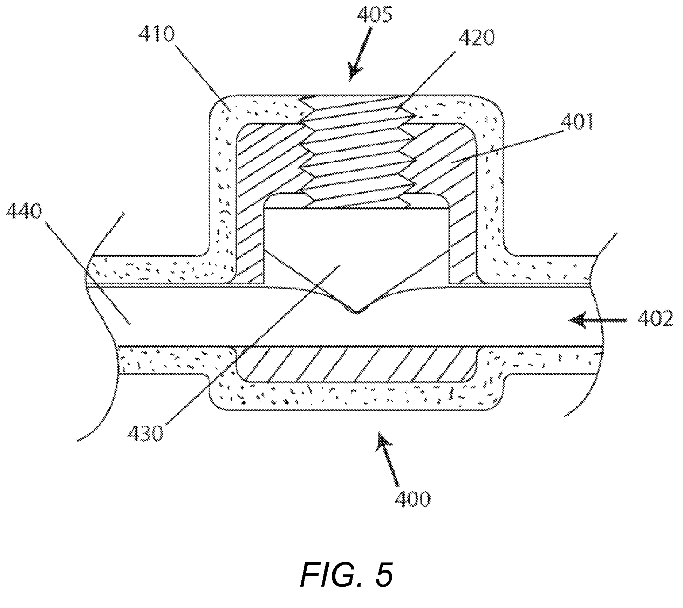

In some embodiments, the fastener 420 engages a pressure plate 430 positioned within the transverse lumen 405. As the fastener 420 is tightened, the pressure plate 430 is moved within the body 401 to obstruct the lead lumen 402. When a lead 440 is placed within the lead lumen 402 and the fastener 420 is tightened, the pressure plate 430 closes down on the lead to keep it in place. As seen in Figures 4A and 5, in some embodiments, the cross section of the pressure plate 430 may be in the shape of a triangle. Alternatively, the cross-section of the pressure plate 430 may be in the shape of a circle, an ovoid, a rectangle, or any other suitable shape. In at least some embodiments, the lead is deformed when the pressure plate 430 is brought into position. In some embodiments, the deformation is slight (e.g., no more than 5% or 10% of the thickness of the lead). In other embodiments, the deformation is more significant (e.g., at least 10% or 25% of the thickness of the lead). The pressure plate 430 may be made of any suitable material, such as, for example, a metal such as titanium, nickel, aluminum, stainless steel, copper, gold, silver, platinum and alloys thereof, or a plastic, rubber or polymer such as polyurethane. In other embodiments (not shown), the fastener 420 directly contacts the lead 440 to lock it in position within the lead anchor 400.

In at least some embodiments, surrounding the body 401, an exterior member 410 may be formed of any suitable biocompatible material. The exterior member 410 may partially or completely surround the body 401. In some embodiments, the exterior member 410 forms a skin around the body 401.

As seen in Figure 4D, the exterior member 410 may also include a septum 451. The septum 451 may comprise silicone. It will be understood that the septum 451 may also be formed of any elastic, biocompatible material including, but not limited to, those suitable for the exterior member 410. In some embodiments, the septum 451 and the exterior member 410 are unitary and formed of the same material. In at least some other embodiments, the septum 451 is a separate member that is attached, glued, fixed, or otherwise coupled to the exterior member 410. The septum 451 may be disposed on the exterior member 410 over the transverse lumen 405 to prevent the set screw from being disengaged from the torque lead anchor 400. In some embodiments, the septum 451 includes a slit 452, or opening to allow a tool to reach the fastener 420. The slit 452 may be large enough to accept the tool, but too small for the fastener 420 to disengage from the torque lock anchor 400.

In at least some embodiments, the exterior member 410 further defines at least one suture element 411. The suture element 411 may be a groove, stub, ridge, eyelet, opening or bore or any other suitable arrangement for suturing the torque lead anchor 400 to the fascia, ligament or other tissue or body structure. The suture element 411 may be positioned anywhere around the circumference of the exterior member 410. In some embodiments, a plurality of suture elements are disposed on the exterior member 410. The exterior member 410 may also define an exterior transverse aperture 412 for receiving the fastener 420 and an exterior lead aperture 413 for receiving the lead.

FIG. 4B is a schematic cross-sectional view of another embodiment of a torque lead anchor with a pressure plate. The torque lead anchor of FIG. 4B includes a body 401 similar to that of FIG. 4A. The body defines a lead lumen 402 and a fastening lumen 421. The torque lead anchor 400 also includes a pressure plate 430. In some embodiments, as the fastener 420 is tightened, the pressure plate is drawn in contact with the lead (not shown) disposed within the lead lumen 402. Thus, when the fastener 420 is tightened, the pressure plate 430 is drawn toward to the body 401, the cross-sectional area of the lead lumen is reduced and the lead is secured. As an example, the fastener 420 and the interior of the fastening lumen 421 of the pressure plate 430 are threaded so that the tightening the fastener 420 draws the pressure plate 430 in contact with the lead. FIG. 4C is a schematic top view of the torque lead anchor 400 of Figure 4B. As can be appreciated from FIG. 4C, the torque lead anchor 400 may be formed with a lower profile so that the lead anchor 400 does not excessively project from the surface of the tissue.

FIG. 5 is a schematic cross-sectional view of the torque lead anchor 400 of Figure 4A after a lead 440 has been inserted in the lead anchor. As can be appreciated from Figure 5, as the fastener 420 is tightened, the pressure plate 430 is brought in contact with the lead 440 within the lead lumen 402. When the fastener 420 is completely tightened, the lead is locked in place within the lead anchor. In some embodiments, the lead 440 is partially deformed when locked in place.

FIG. 6 is a schematic cross-sectional view of another embodiment of a torque lead anchor 600 with a sleeve. As shown in Figure 6, the torque lead anchor 600 comprises a body 601 and an exterior member 610. The body 601 further contains a lead lumen 602 through which a lead may pass. The lead lumen 602 has a first opening 603 and a second opening 604 for insertion of the lead. The body 601 further defines a transverse lumen 605 for accepting a fastener 620. Surrounding the body 601, an exterior member 610 may be formed of a biocompatible material. The exterior member 610 further defines a suture element 611.

As depicted in FIG. 6, in some embodiments, the fastener 620 engages a sleeve 630 positioned within the lead lumen 602. The sleeve 630 may be a substantially hollow cylinder or sheath, and may be made of any suitable material, for example, a metal such as titanium, nickel, aluminum, stainless steel, copper, gold, silver, platinum and alloys thereof, or a plastic, rubber or polymer such as polyurethane. In other embodiments, the fastener 620 directly contacts the lead to lock it in position within the lead anchor. In some embodiments the sleeve 630 is disposed within the body 601 and receives the lead when the lead is passed through the first opening 603 of the lead lumen 602. In another embodiment, the sleeve 630 is removable and is placed around the lead before it is inserted into the lead anchor 600. In some embodiments, the sleeve is deformable when the fastener is tightened. The deformation of the sleeve may be slight (e.g., no more than 5% or 10% of the thickness of the sleeve) or more significant (e.g., at least 10% or 25% of the thickness of the sleeve).

FIG. 7 is a schematic cross-sectional view of the torque lead anchor 600 of Figure 6 after a lead 640 has been inserted in the lead anchor. As the fastener 620 is tightened, the sleeve 630 is deformed and obstructs the lead lumen. When a lead 640 is placed within the lead lumen and the fastener 620 is tightened, the sleeve 630 closes down on the lead to keep it in place. In at least some embodiments, the lead 640 is partially deformed when the sleeve 630 is acted upon by the fastener.

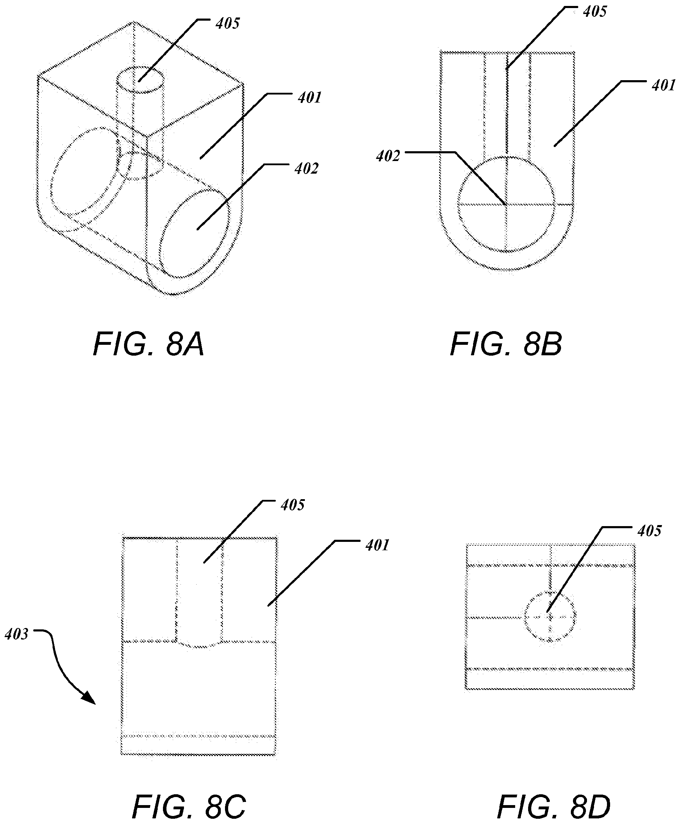

FIGS. 8A-8D are schematic views of a portion of another embodiment of a lead anchor. As illustrated, the body 401 contains a lead lumen 402 for receiving a lead and a transverse lumen 405 for receiving a fastener 420. The lead lumen 402 may have a radius smaller, equal to, or greater than the radius of the transverse lumen 405. In at least some embodiments, the height of the body 401 may be between 0.200 and 0.600 inches (0.508 to 1.52 cm) or between 0.200 and 0.400 inches (0.508 to 1.02 cm). In at least some embodiments, the length of the body 401 may be between 0.200 and 0.400 inches (0.508 to 1.02 cm) or between 0.100 and 0.300 inches (0.254 to 0.762 cm). In at least some embodiments, the width of the body 401 may be between 0.100 and 0.300 inches (0.254 to 0.762 cm) or between 0.200 and 0.500 inches (0.508 to 1.27 cm).

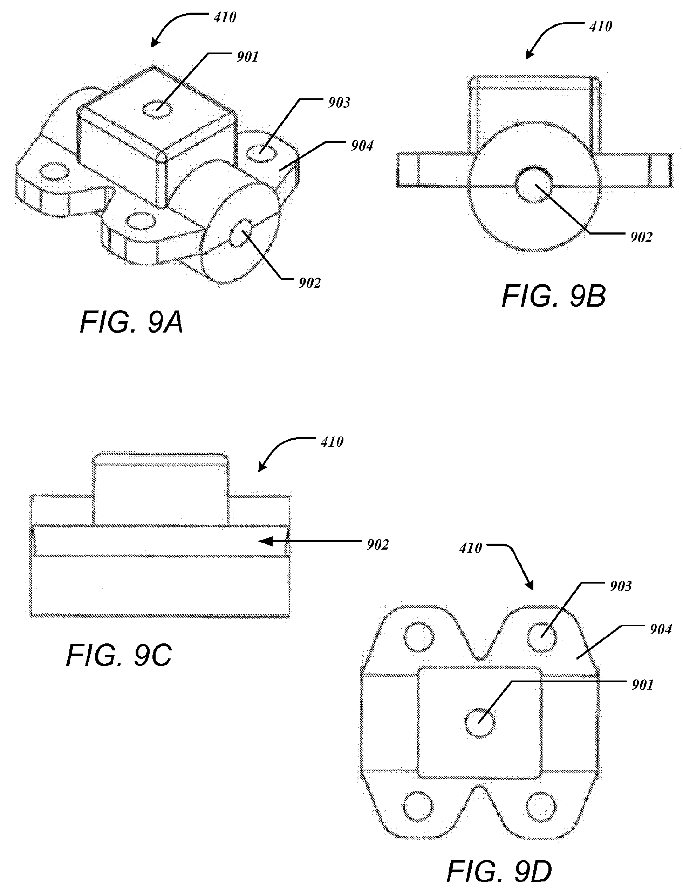

FIGS. 9A-9D are schematic views of a portion of one embodiment of a lead anchor. As can be appreciated from FIG. 9A, the exterior member 410 contains an exterior transverse aperture 901 for receiving a fastener and an exterior lead aperture 902 for receiving a lead. Thus a fastener (not shown) is disposed in the exterior transverse aperture 901 and a lead (not shown) is inserted through the exterior lead aperture 902 and into the lead lumen of the body. As can be appreciated from FIGS. 9A, 9B and 9C, the lead anchor may also contain one or more suture tabs 904 extending from the exterior member 410 and suture eyelets 903 formed in the suture tabs for suturing the lead anchor to the fascia or ligament. In at least some embodiments, the sutures are wrapped or tied around the anchor using the v-shaped portions of the exterior member.

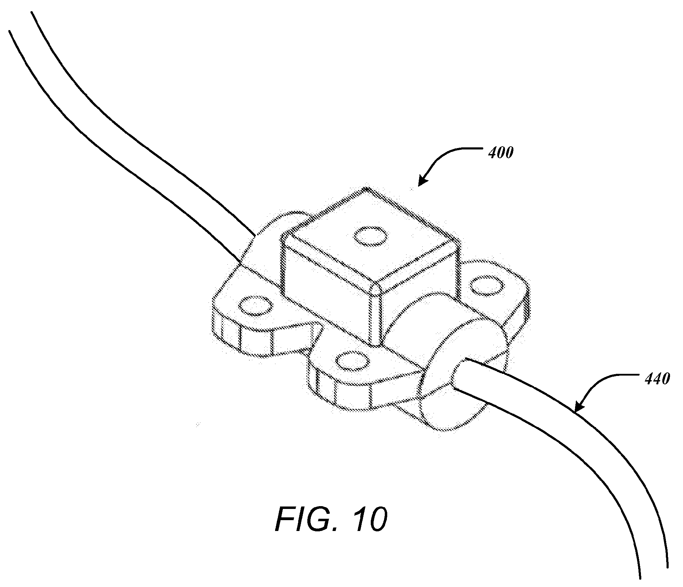

FIG. 10 is a schematic perspective view of the lead anchor 400 of Figure 9A attached to a lead 440. Initially, a lead 440 is placed in position to achieve the desired parathesia at the chosen site of stimulation. In at least some embodiments, the lead is inserted through the exterior lead aperture and into the lead lumen 402 of the body 401. The lead anchor 400 is slid along the length of the lead until it is in the desired position for anchoring to the ligament or fascia. In other embodiments, the body 401, the exterior member 410, or both may be formed of two complementary members that, when joined around a lead 440, form the lead anchor. Thus, no sliding of the lead is necessary. Each of the two complementary members maybe joined together through clasps, buckles, fasteners or by any other suitable arrangement. After the lead has been positioned as desired a fastener is tightened so that the lead 440 is locked in place within the lead anchor 400.

FIG. 11 is a schematic perspective view of the lead anchor 400 of Figure 9A and a tool 1100. The tool 1100 may be a screwdriver, wrench, pliers, or drill or any other suitable tool useful for setting, fixing, screwing, tightening, fastening or fitting a fastener with the lead anchor 400. In at least some embodiments, the tool 1100 is a torque limiting tool set to a certain threshold above which it will no longer tighten the fastener. With this method, over-tightening of the fastener is avoided and the lead can be protected from possible damage due to overtightening the fastener. For example, the torque limiting tool may be configured to limit the number of revolutions or the depth to which a fastener may be advanced.

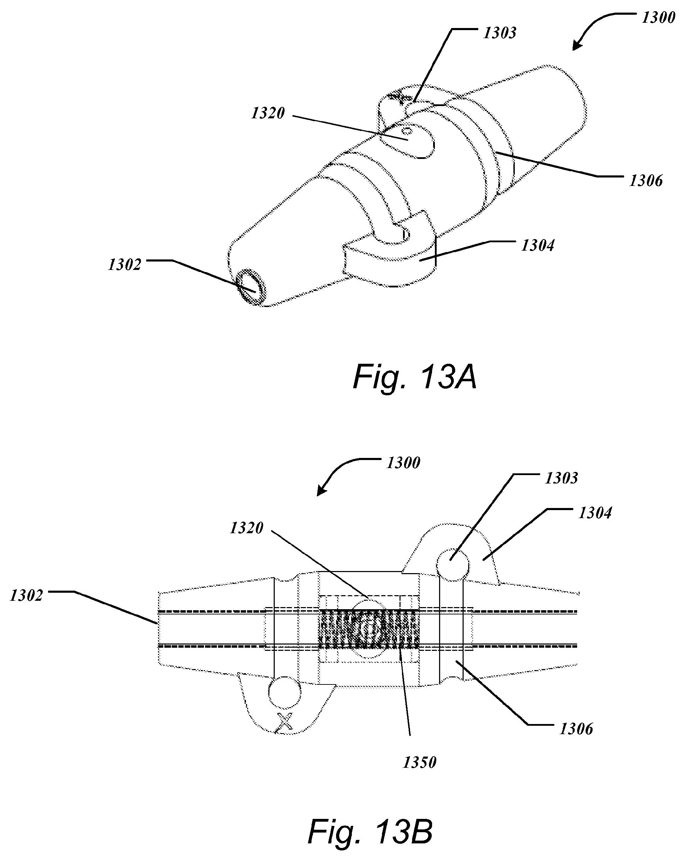

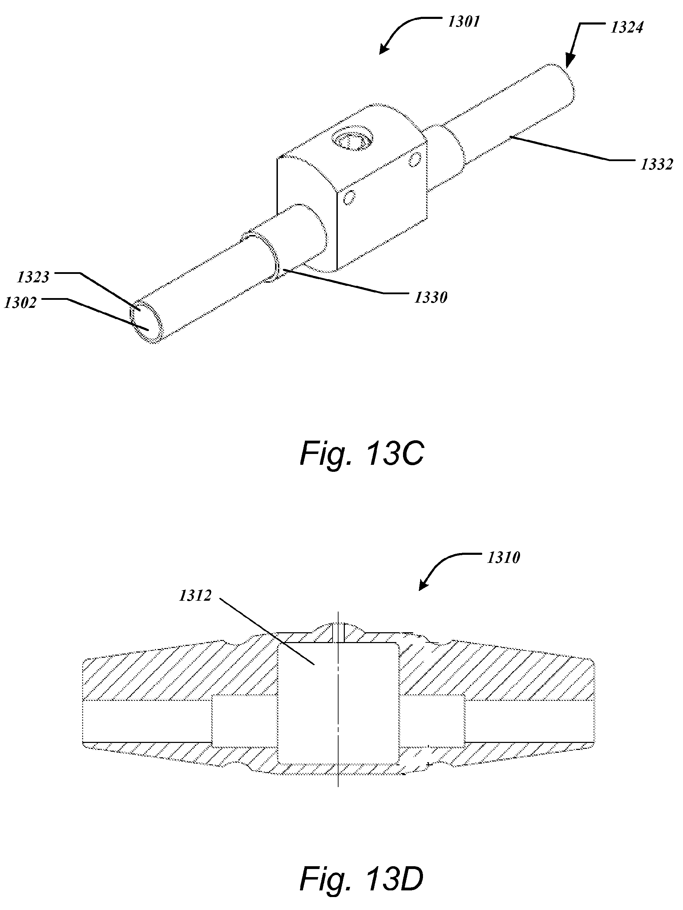

FIGS. 13A to 13D are schematic views of another embodiment of a lead anchor. FIGS. 13A and 13B illustrate the assembled lead anchor 1300. FIG. 13C illustrates a body 1301 of the lead anchor and FIG. 13D is a cross-sectional view of an exterior member 1310 that fits around the body 1301 to form the lead anchor. As can be appreciated from FIG. 13A, the lead anchor 1300 contains an exterior transverse aperture 1305 (FIG. 13C) for receiving a fastener 1320 and an exterior lead aperture 1302 for receiving a lead. Thus a fastener is disposed in the exterior transverse aperture 1305 (FIG. 13C) and a lead (not shown) is inserted through the exterior lead aperture 1302 and into the lead lumen of the body.

As can be appreciated from FIGS. 13A and 13B, the lead anchor 1300 may also contain one or more suture tabs 1304 extending from the lead anchor 1300 (e.g., from the exterior member 1310) and suture eyelets 1303 in the suture tabs for suturing the lead anchor to the fascia or ligament. The suture tabs 1304 can be placed in any suitable arrangement around the lead anchor. For example, one or more suture tabs 1304 can extend from opposite sides of the lead anchor (see, e.g., FIGS. 13A and 9A). Suture tabs extending from opposite sides of the lead anchor can be arranged inany manner including, but not limited to, directly across from each other (see, e.g., FIG. 9A) or in a staggered arrangement (see, e.g., FIG. 13A), for example, with one suture tab extending from a distal region of one side of the lead anchor and another suture tab extending from a proximal region of the opposing side of the lead anchor as illustrated in FIGS. 13A and 13B. The exterior member 1310 may also include one or more suture channels 1306 within which a suture can be wrapped around the lead anchor 1300. It will be understood that suture tabs, suture eyelets, and suture channels can be used with any of the lead anchor embodiments described herein.

As shown in FIGS. 13C and 13D, the torque lead anchor 1300 may include a body 1301 and an exterior member 1310. The body 1301 may be made of a metal, such as titanium, nickel, aluminum, stainless steel, copper, gold, silver, platinum and alloys thereof or any other biocompatible metal, or a rigid plastic or polymer material. The exterior member 1310 includes a space 1312 for receiving the body 1301. The exterior member 1310 may be formed of any biocompatible material such as plastics and polymers including, but not limited to, silicone, polyvinyl chloride, fluoropolymers, polyurethane, polycarbonate, acrylic compounds, thermoplastic polyesters, polypropylene, low-density polyethylenes, and other thermoplastic elastomers. In at least some embodiments, the exterior member 1310 is made of silicone.

The body 1301 may contain a lead tube 1332 with a lead lumen 1302 through which a lead may pass. The lead tube 1332 has a first opening 1323 and a second opening 1324 for insertion of the lead. The lead tube 1332 may also include ridges 1350 (FIG. 3B), which may be concentric or spiraling, for better engagement with the lead. The lead tube 1332 may alternatively define an interior thread or another pattern for better engagement with the lead. The lead tube 1332 may be made of a metal, such as titanium, nickel, aluminum, stainless steel, copper, gold, silver, platinum and alloys thereof or any other biocompatible metal, or a rigid plastic or polymer material.

Optionally, the body 1301 includes a sleeve 1330. The sleeve 1330 may be a substantially hollow cylinder or sheath, and may be made of any suitable material, for example, a metal such as titanium, nickel, aluminum, stainless steel, copper, gold, silver, platinum and alloys thereof, or a plastic, rubber or polymer such as polyurethane. In operation, when the fastener 1320 is fastened to retain the lead within the lead anchor, the fastener engages the sleeve 1330 positioned within the lead lumen 1302. The sleeve 1330, in turn, engages the lead.

As described above, in at least some embodiments the lead anchor is coupled to the lead by inserting one end of the lead into the lead lumen of the lead anchor and sliding the lead anchor along the length of the lead until the lead anchor is in the desired position for anchoring to patient tissue (e.g., one or more ligaments, fascia, or the like). In some cases, it may be advantageous to couple the lead anchor to the lead without sliding the lead anchor along the length of the lead.

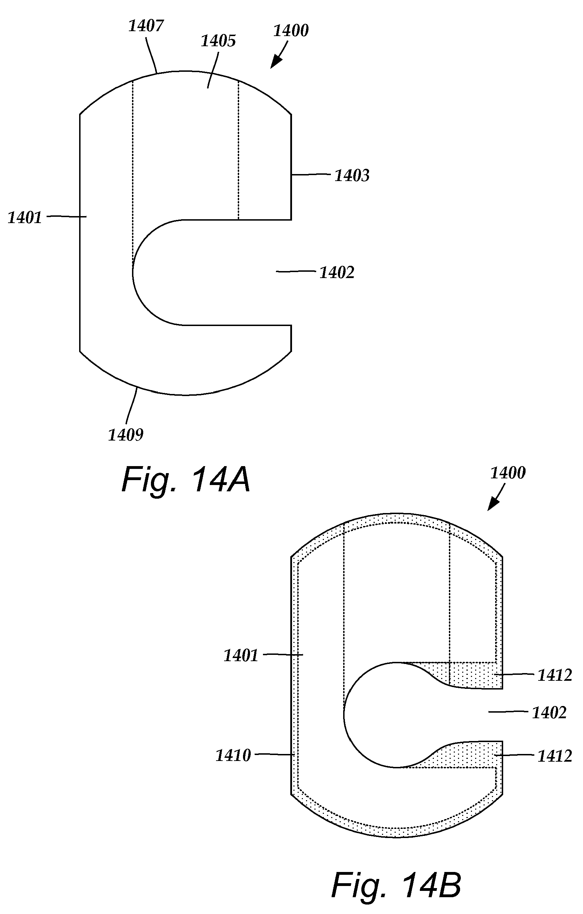

FIGS. 14A to 14C are schematic views of another embodiment of the lead anchor that enable the lead anchor to couple to the lead by sliding the lead anchor laterally onto the lead (*i.e.,* from a side of the lead). In at least some cases, the lead anchor can be slid laterally onto the lead without sliding the lead anchor longitudinally along the length of the lead from one end of the lead.

FIG. 14A is a schematic end view of another embodiment of a lead anchor 1400. The lead anchor 1400 includes a body 1401 that defines a lead slot 1402 and a transverse lumen 1405. The lead slot 1402 extends along an outer surface of a front side 1403 of the body 1401 such that a longitudinal axis of the lead slot 1402 is open along the front side 1403 of the body 1401. The transverse lumen 1405 extends along an interior portion of the body 1401 from an outer surface, such as a top portion 1407, of the body 1401 and intersects with the lead slot 1402. In alternate embodiments, the transverse lumen 1405 extends from other outer portions of the body 1401, such as a bottom portion 1409 of the body 1401.

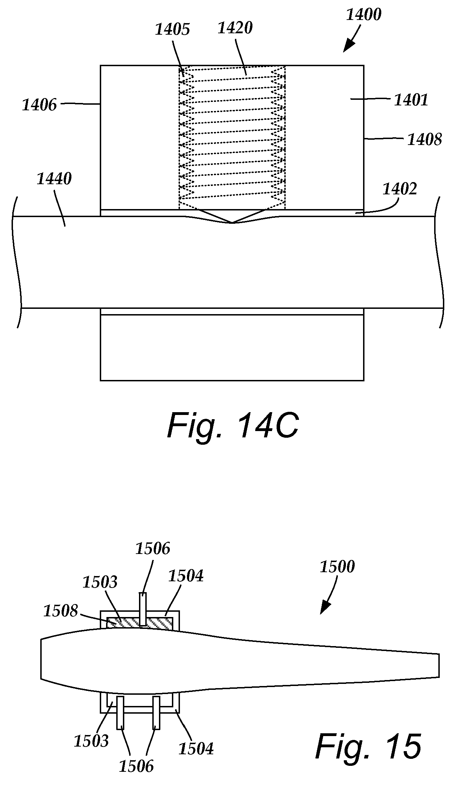

The lead anchor 1400 may include an exterior member, as described in detail above. FIG. 14B is a schematic side view of an embodiment of the lead anchor 1402 having an external member 1410 disposed over the outer surfaces of the body 1401. In some cases, the external member 1410 may form a rim (or lip) 1412 along at least a portion of the opening of the lead slot 1402. In Figure 14B, the external member 1410 is shown forming the lip 1412 along both edges of the opening of the lead slot 1402. The lip 1412 reduces the diameter of the opening of the lead slot 1402. Such a configuration may facilitate containment of the lead 1440 within the lead slot 1402. FIG. 14C illustrates a front view of the lead anchor 1400 with a lead 1440 disposed in the lead slot 1402. As shown in FIG. 14C, the lead slot 1402 extends from a first end 1406 of the body 1401 to a second end 1408 of the body 1402 opposite to the first side 1406.

The lead anchor 1400 is configured and arranged such that the lead anchor 1400 can be coupled to the lead 1440 laterally by passing the lead slot 1402 of the lead anchor 1400 over a portion of the lead 1440 disposed in proximity to a desired anchoring location (e.g., patient tissue). Thus, the lead anchor 1400 can be coupled to the lead 1440 without passing the lead anchor 1400 through an end of the lead 1440 and "threading" the lead anchor 1400 to a portion of the lead 1440 disposed in proximity to a desired anchoring location.

In some embodiments, the body 1401 contains more than one lead slot 1402 so that the lead anchor 1400 is able to be placed over more than one lead 1440. The lead slot 1402 walls may be a friction fit with the lead 1440 or can be large enough to allow the lead 1440 to pass through freely. In at least some embodiments (see *e.g.,* FIG. 4D), the lead lumen 402 may also include ridges, which may be concentric, for better engagement with the lead 1440. The lead slot 1402 may alternatively define an interior thread or another pattern for better engagement with the lead 1440.

As also shown in FIGS. 14A-14C, the transverse lumen 1405 extends from an outer edge of the body 1401 to the lead slot 1402. The transverse lumen 1405 may have a cross-section that is substantially circular. In other embodiments, the body 1401 defines a transverse lumen 1405 with a cross-section in the shape of a triangle, a square, an ovoid, or any other suitable shape. In some embodiments, the transverse lumen 1405 is positioned perpendicular to the longitudinal axis of the lead slot 1402. In other embodiments, the transverse lumen 1405 may be defined so a longitudinal axis of the transverse lumen 1405 engages the lead 1440 within the lead lumen 1402 at a 15, 30, or 45 degree angle or any other suitable angle with respect to the longitudinal axis of the lead slot 1402.

In some embodiments, the transverse lumen 1405 intersects the lead slot 1402 and extends through it so that a cross-shaped void is formed through the body 1401. In at least some embodiments, the transverse lumen 1405 merges with the lead slot 1402 but does not extend through it, so that the cross-section of the body 1401 defines a T-shaped bore. In at least some embodiments, the lead slot 1402 and transverse lumen 1405 intersect with a sleeve or a plate disposed between the two (see, *e.g.,* FIGS. 6 and 7).

As will be appreciated, the transverse lumen 1405 can be configured and arranged to receive a fastener 1420. In embodiments with multiple lead slots 1402, the body 1401 may define more than one transverse lumen 1405 for receiving a plurality of fasteners 1420. Additionally, the transverse lumen 1405 may include a thread, groove, crease, channel, duct, or rib for facilitating or accepting the fastener 1420.

The fastener 1420 may be, for example, a pin, clamp, latch, lug, nail, bolt, dowel, rod, rivet, screw or any combination thereof or any other suitable item for engaging and anchoring the lead 1440. The fastener 1420 may engage or couple to the lead anchor 1400 by any method such as, for example, tightening, screwing or pushing. In some embodiments, the fastener 1420 is a set screw with a thread to be received by the transverse lumen 1405 of the body 1401. The set screw may be tightened through the use of the torque limiting tool 1100 (see *e.g.,* FIG. 11). As the fastener 1420 engages the body 1401 through the transverse lumen 1405, the fastener 1420 is brought closer to the lead slot 1402.

In some embodiments, the fastener 1420 engages the pressure plate 430 (see *e.g.,* FIG. 4A) positioned within the transverse lumen 1405. As the fastener 1420 is tightened, the pressure plate 430 is moved within the body 1401 to obstruct the lead lumen 1402. When the lead 1440 is placed within the lead slot 1402 and the fastener 1420 is tightened, the pressure plate 430 closes down on the lead 1440 to keep the lead 1440 in place. In at least some embodiments, the lead is deformed when the pressure plate 430 is brought into position. In some embodiments, the deformation is slight *(e.g.,* no more than 5% or 10% of the thickness of the lead). In other embodiments, the deformation is more significant (e.g., at least 10% or 25% of the thickness of the lead). The pressure plate 430 may be made of any suitable material, such as, for example, a metal such as titanium, nickel, aluminum, stainless steel, copper, gold, silver, platinum and alloys thereof, or a plastic, rubber or polymer such as polyurethane. In other embodiments (not shown), the fastener 420 directly contacts the lead 1440 to lock it in position within the lead anchor 1400.

As described above, in at least some embodiments the lead anchor is secured to patient tissue (e.g., one or more ligaments, fascia, or the like) using sutures. In some cases, it may be difficult to consistently secure the lead anchor to patient tissue using sutures. In at least some embodiments, lead anchors are configured and arranged to enable the lead anchor to be secured to the patient using staples. It will be understood that staples can be used with any of the lead anchor embodiments described herein including, for example, embodiments of lead anchors with lead lumens and embodiments of lead anchors with lead slots.

FIG. 15 is a schematic perspective view of one embodiment of a lead anchor 1500 that includes one or more staple tabs 1504 extending from the lead anchor 1500 and staple eyelets 1503 defined in the staple tabs 1504 for stapling the lead anchor 1500 to the patient tissue (e.g., fascia or ligament). Staples 1506 are secured to the lead anchor 1500 by passing a portion of the staples 1506 through the staple eyelets 1503 of the stable tabs 1504. The staple tabs 1504 can have any suitable number of staple eyelets 1503, and the staple eyelets 1503 can be configured and arranged to receive any suitable number of staples 1506. For example, in FIG. 15 one of the staple eyelets 1503 is shown receiving a single staple 1506, while another of the staple eyelets 1503 is shown receiving a plurality of staples 1506.

Optionally, at least a portion of one or more of the staple eyelets 1503 may include a meshed matrix 1508 through which the staple 1506 may pierce. In some cases, the outer member (see *e.g.,* 1410 in FIG. 14B) may be disposed over all, or a portion, of the staple tabs 1504 and the staple eyelets 1503. In which case, it may be useful to include the mesh matrix 1508, for example, to prevent tearing of the external member when the staple 1506 extends through the staple eyelet 1503. The mesh matrix 1508 can be formed from any suitable material including, for example, polyethylene terephthalate, or the like.

The staple tabs 1504 can be placed in any suitable arrangement around the lead anchor 1500. For example, one or more staple tabs 1504 can extend from opposite sides of the lead anchor (see *e.g.,* FIGS. 13A and 9A). Staple tabs 1504 extending from opposite sides of the lead anchor 1500 can be arranged in any manner including, but not limited to, directly across from each other (see *e.g.,* FIG. 9A) or in a staggered arrangement (see *e.g.,* FIG. 13A), for example, with one staple tab 1504 extending from a distal region of one side of the lead anchor 1500 and another staple tab 1504 extending from a proximal region of the opposing side of the lead anchor 1500, as illustrated in FIGS. 13A and 13B. It will be understood that staple tabs 1504 and staple eyelets 1503 can be used with any of the lead anchor embodiments described herein.

In some cases, the lead anchor may include one or more elongated, tapered ends. It may be useful to design the lead anchor with one or more elongated, tapered ends, especially on a distal end of the lead anchor, to facilitate insertion of the lead anchor into the patient. It will be understood that elongated, tapered ends can be used with any of the lead anchor embodiments described herein including, for example, embodiments of lead anchors with lead lumens and embodiments of lead anchors with lead slots.

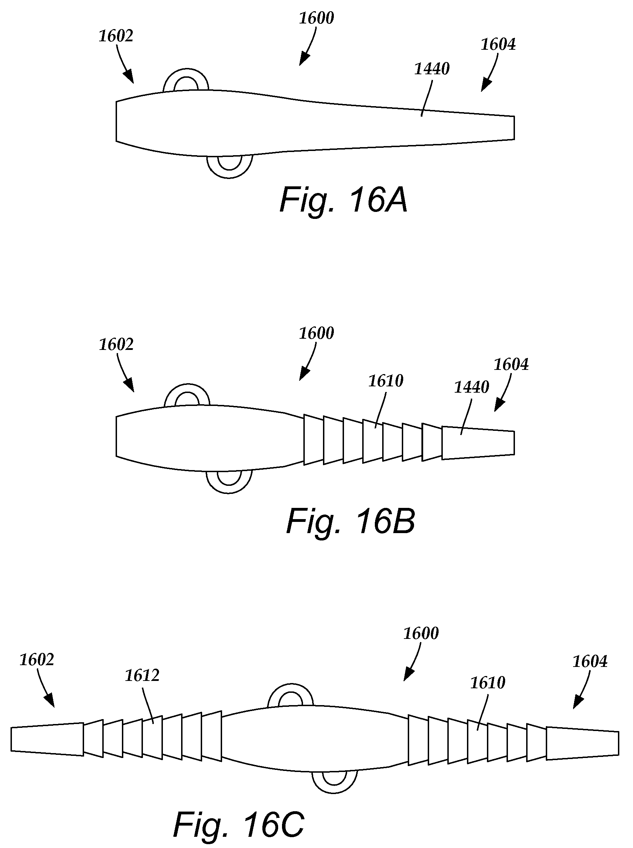

FIG. 16A is a schematic side view of one embodiment of a lead anchor 1600 having a proximal end 1602 and a distal end 1604. In FIG. 16A, the distal end 1604 of the lead anchor 1600 is shown having an elongated, tapered shape. During insertion of the lead anchor 1600, a distally-directed force applied to the lead anchor 1600 by a medical practitioner may be countered, in part, by resistance from patient tissue. When the distal end of the lead anchor 1600 has an elongated, tapered shape, the amount of resistance from patient tissue may be reduced.

Additionally, during implantation of the lead anchor 1600, there may be a tendency for patient tissue to apply a proximally-directed force against the distal end of the lead anchor 1600, thereby "pushing" the lead anchor proximally towards the point of entry into the patient. In which case, it may be advantageous to form the elongated, tapered distal end 1604 with one or more ridges, or barbs, configured and arranged to oppose a proximally-directed force, thereby counteracting at least some of the force applied by patient tissue.

FIG. 16B is a schematic side view of one embodiment of the lead anchor 1600 having a plurality of ridges, such as ridge 1610, configured to at least partially counteract proximally-directed forces applied by patient tissue during implantation of the lead anchor 1600. In at least some embodiments, the ridges 1610 have diameters that taper in diameter moving distally along the lead anchor 1600.

In some cases, it may be useful to provide oppositely-directed ridges in addition to, or in lieu of, the ridges shown in FIG. 16B. When oppositely-directed ridges are implemented, the lead anchor may be more inclined to maintain its position within the patient and be less likely to move either distally or proximally in response to one or more forces caused by patient tissue. In some cases, the oppositely-directed ridges are disposed on the proximal end of the lead anchor.

FIG. 16C is a schematic side view of one embodiment of the lead anchor 1600 having a plurality of first ridges, such as first ridge 1610, disposed on the distal end 1604 of the lead anchor 1600, and a plurality of second ridges, such as second ridge 1612, disposed on the proximal end 1602 of the lead anchor 1600. The number of first ridges 1610 can be less than, equal to, or greater than the number of second ridges 1612. It will be understood that one or more ridges can be used with any of the lead anchor embodiments described herein including, for example, embodiments of lead anchors with lead lumens and embodiments of lead anchors with lead slots.

Magnetic resonance imaging ("MRI") is commonplace in many medical settings. Conventional implanted electrical stimulation systems are often incompatible MRI due to large radio frequency ("RF") pulses used by MRI devices. The RF pulses can generate transient signals in the conductors and electrodes of an implanted lead. These signals can have deleterious effects including, for example, unwanted heating of the tissue causing tissue damage, induced currents in the lead, or premature failure of electronic components. It may be useful to form a lead anchor such that it is compatible with MRI devices, as well as other devices that potentially expose a patient to RF irradiation.

A common cause of the electrical interaction between the electrical stimulation system and RF irradiation is common-mode coupling of the applied electromagnetic field. The interaction can be modeled as a series of distributed sources along the elongated conductive structures of the electrical stimulation system, such as leads, lead extensions, or conductors within leads or lead extensions. Common-mode induced RF currents may reach amplitudes of greater than one ampere in MRI environments. Such currents can cause heating and potentially disruptive voltages within electronic circuits.

To reduce the susceptibility of the electrical stimulation system to undesired RF irradiation, it may be advantageous to construct any of the above-mentioned embodiments of the lead anchor housing from one or more materials that reduce susceptibility of the lead anchor to undesired RF irradiation, while still maintaining appropriate biocompatibility for prolonged implantation and sufficient mechanical integrity to anchor a lead. Some exemplary materials include one or more polymers (e.g., polyetheretherketone, polyethersulfone, polyethylene, polypropylene, polyurethane, polyetherimide, polycarbonate, nylon, polysulfone, polymethylmethacrylate, or the like or combinations thereof), one or more ceramics, one or more non-magnetically reactive metals, or the like or combinations thereof.

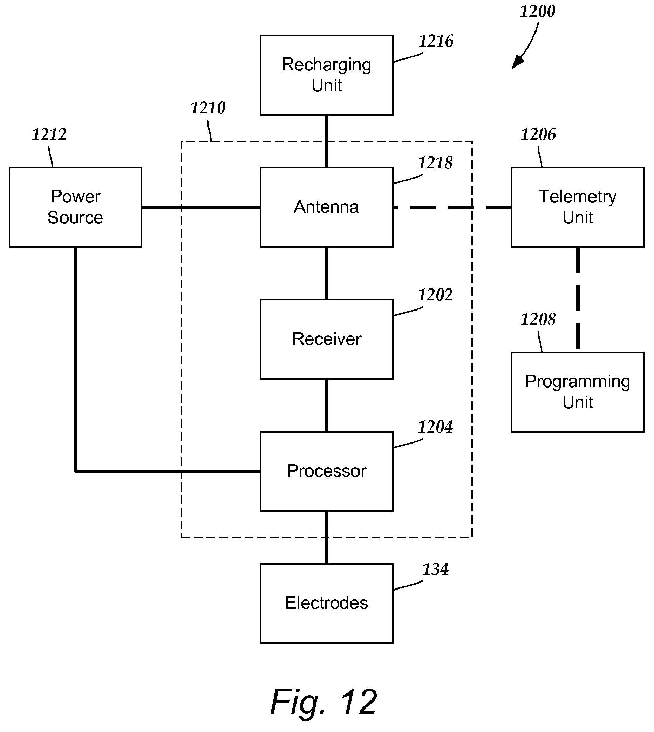

Figure 12 is a schematic overview of one embodiment of components of an electrical stimulation system 1200 including an electronic subassembly 1210 disposed within a control module. It will be understood that the electrical stimulation system can include more, fewer, or different components and can have a variety of different configurations including those configurations disclosed in the stimulator references cited herein.

Some of the components (for example, power source 1212, antenna 1218, receiver 1202, and processor 1204) of the electrical stimulation system can be positioned on one or more circuit boards or similar carriers within a sealed housing of an implantable pulse generator, if desired. Any power source 1212 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent No. 7,437,193.

As another alternative, power can be supplied by an external power source through inductive coupling via the optional antenna 1218 or a secondary antenna. The external power source can be in a device that is mounted on the skin of the user or in a unit that is provided near the user on a permanent or periodic basis.

If the power source 1212 is a rechargeable battery, the battery may be recharged using the optional antenna 1218, if desired. Power can be provided to the battery for recharging by inductively coupling the battery through the antenna to a recharging unit 1016 external to the user. Examples of such arrangements can be found in the references identified above.

In one embodiment, electrical current is emitted by the electrodes 134 on the paddle or lead body to stimulate nerve fibers, muscle fibers, or other body tissues near the electrical stimulation system. A processor 1204 is generally included to control the timing and electrical characteristics of the electrical stimulation system. For example, the processor 1204 can, if desired, control one or more of the timing, frequency, strength, duration, and waveform of the pulses. In addition, the processor 1204 can select which electrodes can be used to provide stimulation, if desired. In some embodiments, the processor 1204 may select which electrode(s) are cathodes and which electrode(s) are anodes. In some embodiments, the processor 1204 may be used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 1208 that, for example, allows modification of pulse characteristics. In the illustrated embodiment, the processor 1204 is coupled to a receiver 1202 which, in turn, is coupled to the optional antenna 1218. This allows the processor 1204 to receive instructions from an external source to, for example, direct the pulse characteristics and the selection of electrodes, if desired.

In one embodiment, the antenna 1218 is capable of receiving signals (e.g., RF signals) from an external telemetry unit 1206 which is programmed by a programming unit 1208. The programming unit 1208 can be external to, or part of, the telemetry unit 1206. The telemetry unit 1206 can be a device that is worn on the skin of the user or can be carried by the user and can have a form similar to a pager, cellular phone, or remote control, if desired. As another alternative, the telemetry unit 1206 may not be worn or carried by the user but may only be available at a home station or at a clinician's office. The programming unit 1208 can be any unit that can provide information to the telemetry unit 1206 for transmission to the electrical stimulation system 1200. The programming unit 1208 can be part of the telemetry unit 1206 or can provide signals or information to the telemetry unit 1206 via a wireless or wired connection. One example of a suitable programming unit is a computer operated by the user or clinician to send signals to the telemetry unit 1206.

The signals sent to the processor 1204 via the antenna 1218 and receiver 1202 can be used to modify or otherwise direct the operation of the electrical stimulation system. For example, the signals may be used to modify the pulses of the electrical stimulation system such as modifying one or more of pulse duration, pulse frequency, pulse waveform, and pulse strength. The signals may also direct the electrical stimulation system 1200 to cease operation, to start operation, to start charging the battery, or to stop charging the battery. In other embodiments, the stimulation system does not include an antenna 1218 or receiver 1202 and the processor 1204 operates as programmed.

Optionally, the electrical stimulation system 1200 may include a transmitter (not shown) coupled to the processor 1204 and the antenna 1218 for transmitting signals back to the telemetry unit 1206 or another unit capable of receiving the signals. For example, the electrical stimulation system 1200 may transmit signals indicating whether the electrical stimulation system 1200 is operating properly or not or indicating when the battery needs to be charged or the level of charge remaining in the battery. The processor 1204 may also be capable of transmitting information about the pulse characteristics so that a user or clinician can determine or verify the characteristics.

The above specification, examples and data provide a description of the manufacture and use of the composition of the invention. Since many embodiments of the invention can be made without departing from the scope of the invention, the invention also resides in the claims hereinafter appended.

## Claims

1. A lead anchor (400; 600; 1300; 1400; 1500; 1600) comprising:
a body (401; 601; 1301; 1401) having an outer surface, a top end, a front side, a first end, and a second end opposite to the first end, the body defining
a lead slot (1402) configured and arranged to receive a lead, the lead slot (1402) defining an elongated opening extending along the front side of the body from the first end to the second end, and
a transverse lumen (1405) extending from the top end of the body and intersecting the lead slot (1402);
an exterior member (410; 610; 1310) disposed around at least a portion of the body (401; 601; 1301; 1401), the exterior member being formed of a biocompatible material; and
a fastener (1420) disposed in the transverse lumen (1405), the fastener configured and arranged for fastening the received lead to the lead anchor by deforming a portion of the lead.

2. The lead anchor of claim 1, wherein the exterior member (410; 610; 1310; 1410) comprises a lip (1412), the lip extending along at least a portion of the lead slot opening.

3. The lead anchor of either claim 1 or claim 2, further comprising at least one staple tab (1504) extending from the lead anchor, the at least one staple tab configured and arranged for receiving at least one staple to staple the lead anchor to patient tissue.

4. The lead anchor of claim 3, wherein the at least one staple tab (1504) forms at least one staple eyelet through which a portion of the at least one staple is configured and arranged to extend when the at least one staple staples the lead anchor to patient tissue.

5. The lead anchor of claim 4, further comprising at least one mesh matrix (1508) disposed over the at least one staple eyelet such that when the at least one staple staples the lead anchor to patient tissue the at least one staple extends through the at least one mesh matrix.

6. The lead anchor of any one of claims 1-5, wherein the first end has an elongated, tapered shape.

7. The lead anchor of claim 6, wherein the first end comprises at least one ridge having a diameter that tapers as the at least one ridge extends away from the second end of the lead anchor.

8. The lead anchor of any one of claims 1-7, wherein the first end and the second end each have elongated, tapered shapes.

9. The lead anchor of claim 8, wherein the first end comprises at least one first ridge and the second end comprises at least one second ridge, the at least first one ridge having a diameter that tapers as the at least one first ridge extends away from the second end of the lead anchor, and the at least one second ridge having a diameter that tapers as the at least one second ridge extends away from the first end of the lead anchor.

10. The lead anchor of any one of claims 1-9, wherein the body(401; 601; 1301; 1401) comprises at least one of a polymer or a ceramic.

11. The lead anchor of any one of claims 1-10, wherein the body (401; 601; 1301; 1401) comprises at least one non-magnetically reactive metal.

12. An implantable stimulation device, comprising:
a lead (308; 334; 440; 640; 1440) having an electrode array; and
the lead anchor (400; 600; 1300; 1400; 1500; 1600) of any one of claims 1-11.

13. The implantable stimulation device of claim 12, further comprising a control module coupleable to the lead, wherein optionally the implantable stimulation device is a spinal cord stimulator.

## Patentansprüche

1. Leitungsanker (400; 600; 1300; 1400; 1500; 1600) der aufweist:
einen Körper (401; 601; 1301; 1401), der eine Außenfläche, ein oberes Ende, eine Vorderseite, ein erstes Ende und ein zweites Ende gegenüber dem ersten Ende aufweist,
wobei der Körper definiert:
einen Leitungsspalt (1402), der konfiguriert und angeordnet ist, eine Leitung aufzunehmen, wobei der Leitungsspalt (1402) eine längliche Öffnung definiert, die sich entlang der Vorderseite des Körpers vom ersten Ende zum zweiten Ende erstreckt, und
ein transversales Lumen (1405), das sich vom oberen Ende des Körpers erstreckt und der Leitungsspalt (1402) schneidet;
ein äußeres Element (410; 610; 1310), das um mindestens einen Abschnitt des Körpers (401; 601; 1301; 1401) angeordnet ist, wobei das äußere Element aus einem biokompatiblen Material ausgebildet ist; und
ein Befestigungselement (1420), das im transversalen Lumen (1405) angeordnet ist, wobei das Befestigungselement zum Befestigen der aufgenommenen Leitung am Leitungsanker durch Verformen eines Abschnitts der Leitung konfiguriert und angeordnet ist.

2. Leitungsanker nach Anspruch 1, wobei das äußere Element (410; 610; 1310; 1410) einen Ansatz (1412) aufweist, wobei sich der Ansatz längs mindestens eines Abschnitts der Leitungsspaltöffnung erstreckt.

3. Leitungsanker nach entweder Anspruch 1 oder Anspruch 2, der ferner mindestens eine Klammerlasche (1504) aufweist, die sich vom Leitungsanker erstreckt, wobei die mindestens eine Klammerlasche zum Aufnehmen von mindestens einer Klammer konfiguriert und angeordnet ist, um den Leitungsanker an ein Patientengewebe zu klammern.

4. Leitungsanker nach Anspruch 3, wobei die mindestens eine Klammerlasche (1504) mindestens eine Klammeröse bildet, wobei ein Abschnitt der mindestens einen Klammer so konfiguriert und angeordnet ist, dass er sich durch sie erstreckt, wenn die mindestens eine Klammer den Leitungsanker an Patientengewebe klammert.

5. Leitungsanker nach Anspruch 4, der ferner mindestens eine Maschenmatrix (1508) aufweist, die über der mindestens einen Klammeröse angeordnet ist, so dass sich, wenn die mindestens eine Klammer den Leitungsanker an Patientengewebe klammert, die mindestens eine Klammer durch die mindestens eine Maschenmatrix erstreckt.

6. Leitungsanker nach einem der Ansprüche 1 bis 5, wobei das erste Ende eine längliche, angeschrägte Form aufweist.

7. Leitungsanker nach Anspruch 6, wobei das erste Ende mindestens einen Grat aufweist, der einen Durchmesser aufweist, der konisch zuläuft, wenn sich der mindestens eine Grat vom zweiten Ende des Leitungsankers weg erstreckt.

8. Leitungsanker nach einem der Ansprüche 1 bis 7, wobei das erste Ende und das zweite Ende jeweils längliche, angeschrägte Formen aufweisen.

9. Leitungsanker nach Anspruch 8, wobei das erste Ende mindestens einen ersten Grat aufweist und das zweite Ende mindestens einen zweiten Grat aufweist, wobei der mindestens eine erste Grat einen Durchmesser aufweist, der konisch zuläuft, wenn sich der mindestens eine erste Grat vom zweiten Ende des Leitungsankers weg erstreckt, und der mindestens eine zweite Grat einen Durchmesser aufweist, der konisch zuläuft, wenn sich der mindestens eine zweite Grat vom ersten Ende des Leitungsankers weg erstreckt.

10. Leitungsanker nach einem der Ansprüche 1 bis 9, wobei der Körper (401; 601; 1301; 1401) ein Polymer und/oder eine Keramik aufweist.

11. Leitungsanker nach einem der Ansprüche 1 bis 10, wobei der Körper (401; 601; 1301; 1401) mindestens ein nicht magnetisch reaktives Metall aufweist.

12. Implantierbare Stimulationsvorrichtung, die aufweist:
eine Leitung (308; 334; 440; 640; 1440), die eine Elektrodenanordnung aufweist; und
den Leitungsanker (400; 600; 1300; 1400; 1500; 1600) nach einem der Ansprüche 1 bis 11.

13. Implantierbare Stimulationsvorrichtung nach Anspruch 12, die ferner ein an die Leitung koppelbares Steuermodul aufweist, wobei die implantierbare Stimulationsvorrichtung optional ein Rückenmarkstimulator ist.

## Revendications

1. Ancrage (400 ; 600 ; 1300 ; 1400 ; 1500 ; 1600) de dérivation, comprenant :
un corps (401 ; 601 ; 1301 ; 1401) présentant une surface extérieure, une extrémité supérieure, une face avant, une première extrémité, et une deuxième extrémité opposée à la première extrémité, ledit corps définissant
une fente (1402) de dérivation prévue et agencée pour recevoir une dérivation, ladite fente (1402) de dérivation définissant une ouverture oblongue s'étendant le long de la face avant du corps, de la première extrémité à la deuxième extrémité, et
une lumière transversale (1405) s'étendant depuis l'extrémité supérieure du corps et intersectant la fente (1402) de dérivation ;
un élément extérieur (410 ; 610 ; 1310) disposé autour d'au moins une partie du corps (401 ; 601 ; 1301 ; 1401), ledit élément extérieur étant constitué d'un matériau biocompatible ; et
une fixation (1420) disposée dans la lumière transversale (1405), ladite fixation étant prévue et agencée pour fixer la dérivation reçue à l'ancrage de dérivation par déformation d'une partie de la dérivation.

2. Ancrage de dérivation selon la revendication 1, où l'élément extérieur (410 ; 610 ; 1310 ; 1410) présente une lèvre (1412), ladite lèvre s'étendant le long d'au moins une partie de l'ouverture de fente de dérivation.

3. Ancrage de dérivation selon la revendication 1 ou la revendication 2, comprenant en outre au moins une patte d'agrafe (1504) s'étendant depuis l'ancrage de dérivation, ladite au moins une patte d'agrafe étant prévue et agencée pour recevoir au moins une agrafe afin d'agrafer l'ancrage de dérivation au tissu du patient.

4. Ancrage de dérivation selon la revendication 3, où ladite au moins une patte d'agrafe (1504) forme au moins un oeillet d'agrafe, au travers duquel une partie de ladite au moins une agrafe est prévue et agencée pour s'étendre, quand ladite au moins une agrafe agrafe l'ancrage de dérivation au tissu du patient.

5. Ancrage de dérivation selon la revendication 4, comprenant en outre au moins une grille en treillis (1508) disposée au-dessus dudit au moins un oeillet d'agrafe, de telle manière que, quand ladite au moins une agrafe agrafe l'ancrage de dérivation au tissu du patient, ladite au moins une agrafe traverse ladite au moins une grille en treillis.

6. Ancrage de dérivation selon l'une des revendications 1 à 5, où la première extrémité a une forme oblongue chanfreinée.

7. Ancrage de dérivation selon la revendication 6, où la première extrémité présente au moins une crête dont le diamètre diminue à mesure que ladite au moins une crête s'éloigne de la deuxième extrémité de l'ancrage de dérivation.

8. Ancrage de dérivation selon l'une des revendications 1 à 7, où la première extrémité et la deuxième extrémité ont chacune une forme oblongue chanfreinée.

9. Ancrage de dérivation selon la revendication 8, où la première extrémité présente au moins une première crête et la deuxième extrémité présente au moins une deuxième crête, ladite au moins première une crête ayant un diamètre diminuant à mesure que ladite au moins une première crête s'éloigne de la deuxième extrémité de l'ancrage de dérivation, et ladite au moins une deuxième crête ayant un diamètre diminuant à mesure que ladite au moins une deuxième crête s'éloigne de la première extrémité de l'ancrage de dérivation.

10. Ancrage de dérivation selon l'une des revendications 1 à 9, où le corps (401 ; 601 ; 1301 ; 1401) est constitué de polymère et/ou de céramique.

11. Ancrage de dérivation selon l'une des revendications 1 à 10, où le corps (401 ; 601 ; 1301 ; 1401) comprend au moins un métal réactif non magnétiquement.

12. Dispositif de stimulation implantable, comprenant :
une dérivation (308 ; 334 ; 440 ; 640 ; 1440) comportant un réseau d'électrodes ; et
l'ancrage de dérivation (400 ; 600 ; 1300 ; 1400 ; 1500 ; 1600) selon l'une des revendications 1 à 11.

13. Dispositif de stimulation implantable selon la revendication 12, comprenant en outre un module de commande pouvant être relié à la dérivation, ledit dispositif de stimulation implantable étant facultativement un stimulateur de moelle épinière.
